(19) Europäisches Patentamt
European Patent Office
Office européen des brevets

(11) Veröffentlichungsnummer: **0 023 663**
**B1**

# (12) EUROPÄISCHE PATENTSCHRIFT

(45) Veröffentlichungstag der Patentschrift: **16.05.84**

(21) Anmeldenummer: **80104299.5**

(22) Anmeldetag: **22.07.80**

(51) Int. Cl.³: **C 07 H 15/04,** C 07 H 15/18, C 07 H 15/24 // C07D498/04, C07D307/22, C07C101/28

(54) **Verfahren zur Herstellung neuer sauerstoffhaltiger Heterocyclen.**

(30) Priorität: **25.07.79 US 60261**

(43) Veröffentlichungstag der Anmeldung: **11.02.81 Patentblatt 81/6**

(45) Bekanntmachung des Hinweises auf die Patenterteilung: **16.05.84 Patentblatt 84/20**

(84) Benannte Vertragsstaaten: **AT BE CH DE FR GB IT LI NL**

(56) Entgegenhaltungen:
**DE - A - 2 752 115**
**US - A - 4 024 333**

(73) Patentinhaber: **F. HOFFMANN-LA ROCHE & CO. Aktiengesellschaft, CH-4002 Basel (CH)**

(72) Erfinder: **Uskokovic, Milan Radoje, 253 Highland Avenue, Montclair, N.J. (US)**
Erfinder: **Wovkulich, Peter Michael, 124 Rhoda Avenue, Nutley, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 023 663 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung neuer sauerstoffhaltiger Heterocyclen

Die vorliegende Erfindung betrifft ein Verfahren zur Herstellung neuer sauerstoffhaltiger Heterocyclen der allgemeinen Formel

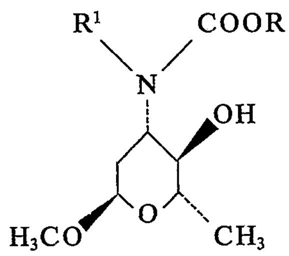

(I)

worin

R $C_{1-7}$-Alkyl,
$R^1$ Wasserstoff oder eine Gruppe $-C(R^2,R^3,R^4)$,
$R^2$ und $R^4$ Wasserstoff, Aryl, Aralkyl oder $C_{1-7}$-Alkyl und
$R^3$ Aryl ist;

deren Enantiomere und Racemate.

Der hier verwendete Ausdruck »$C_{1-7}$-Alkyl« bezeichnet geradkettige oder verzweigte gesättigte aliphatische Kohlenwasserstoffgruppen mit 1—7 C-Atomen, z. B. Methyl, Äthyl, n-Propyl, Isopropyl und Hexyl. $C_{1-7}$-Alkoxy bedeutet Alkoxygruppen mit 1—7 C-Atomen, z. B. Methoxy, Äthoxy oder Isopropoxy. $C_{2-6}$-Alkylen bezeichnet Alkylengruppen mit 2—6 C-Atomen wie Äthylen, Propylen und Butylen. Aryl bezeichnet einkernige aromatische Kohlenwasserstoffgruppen wie Phenyl, welche gegebenenfalls in einer oder mehreren Stellungen durch Halogen, stickstoffhaltige Gruppen, $C_{2-6}$-Alkylendioxy, $C_{1-7}$-Alkyl oder $C_{1-7}$-Alkoxy substituiert sein können. Aryl bezeichnet weiterhin mehrkernige Arylgruppen wie Naphthyl, Anthryl, Phenanthryl, die unsubstituiert oder durch eine oder mehrere der vorgenannten Substituenten substituiert sein können.

Aralkyl bezeichnet einen durch eine Arylgruppe substituierten Alkylrest, insbesondere Benzyl und $C_{1-7}$-Alkyl-substituierte Benzyle, z. B. Cumyl. Der Ausdruck »Halogen« bezieht sich auf Fluor, Chlor, Brom und Jod. Beispiele von Alkalimetallen sind Lithium, Natrium, Kalium und Rubidium. Beispiele von Erdalkalimetallen sind Barium, Magnesium, Calcium und Strontium.

Die Verbindungen der Formel I werden erfindungsgemäß dadurch hergestellt, daß man eine Verbindung der Formel

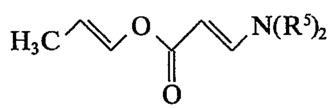

(VI)

worin $R^5$ $C_{1-7}$-Alkyl ist,
mit einer Verbindung der Formel

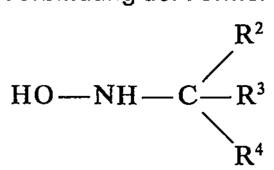

(VII)

worin $R^2$, $R^3$ und $R^4$ die oben angegebene Bedeutung besitzen,
oder einem Salz davon zu einer Verbindung der Formel

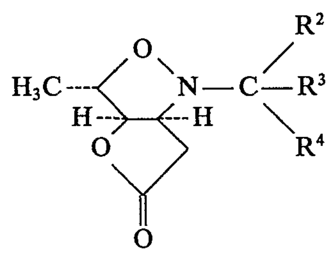

(III)

umsetzt, die man durch Behandlung mit einem geeigneten Reduktionsmittel zu einer Verbindung der Formel

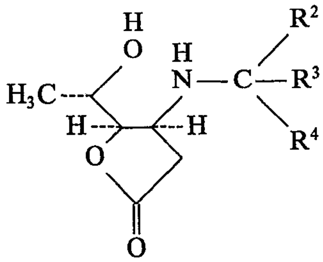

(VIII)

reduziert, welche mit einem Halogenameisensäureester der Formel

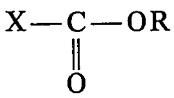

worin

X Halogen ist und
R $C_{1-7}$-Alkyl bedeutet,

in eine Verbindung der Formel

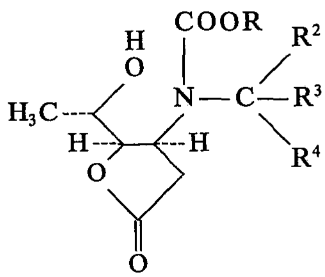

(IX)

übergeführt wird, die ihrerseits durch Behandlung mit einem Reduktionsmittel in eine Verbindung der Formel

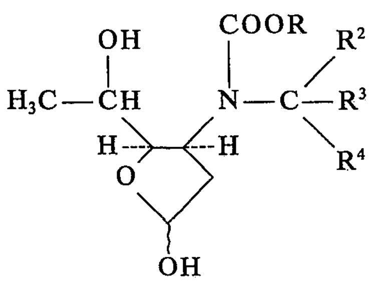

(II)

umgewandelt wird, die man sodann mit Methanol in Gegenwart eines Säurekatalysators zu einer Verbindung der allgemeinen Formel I, in der $R^1$ eine Gruppe $-C(R^2,R^3, R^4)$, darstellt oder einem Enantiomer oder dem Racemat davon umsetzt und gewünschtenfalls die so erhaltene Verbindung hydrogenolysiert, um eine Verbindung der allgemeinen Formel I oder ein Enantiomer oder Racemat davon zu erhalten, in der $R^1$ Wasserstoff ist.

Jedes konventionelle Säureadditionssalz von Verbindungen der Formel VII kann Verwendung finden, beispielsweise von anorganischen Säuren, z. B. Schwefelsäure, Salzsäure und Bromwasserstoffsäure und auch von organischen Säuren wie Oxalsäure, Äpfelsäure und Essigsäure.

Für die Reaktion kann jedes konventionelle inerte organische Lösungsmittel Verwendung finden. Bevorzugte inerte organische Lösungsmittel sind aromatische Kohlenwasserstoffe wie Benzol, Xylol und Toluol. Bei der Reaktion sind Temperatur und Druck nicht kritisch, und die Reaktion kann bei Raumtemperatur und Atmosphärendruck durchgeführt werden. Im allgemeinen wird die Reaktion bei einer Temperatur von 20 bis 15° C ausgeführt, wobei die Rückflußtemperatur des Reaktionsgemisches bevorzugt ist.

Wenn die Verbindung der Formel VII optisch inaktiv ist, erhält man die Verbindung der Formel III als Racemat. Andererseits erhält man aus einer optisch aktiven Verbindung der Formel VII, z. B. der S-Form einer Verbindung der Formel VII, in der $R^3$ die obige Bedeutung hat, $R^4$ Wasserstoff ist und $R^2$ von Wasserstoff und $R^3$ verschieden ist, ein optisch aktives Enantiomer einer Verbindung der Formel

III. Wenn eine Verbindung der Formel VII als Enantiomerengemisch eingesetzt wird, beispielsweise als Gemisch von 80 Gew.-% S- und 20 Gew.-% R-Form, erhält man eine Verbindung der Formel III in einer optischen Reinheit von 80% im Gemisch mit etwa 20% des anderen Enantiomeren. Dieses Enantiomerengemisch kann in den nachfolgenden Reaktionsschritten eingesetzt werden oder mit bekannten Mitteln aufgetrennt werden. Von den Verbindungen der Formel VII sind die optisch aktiven Verbindungen, insbesondere das S-Enantiomere, bevorzugt.

Die Verbindung der Formel III wird in eine Verbindung der Formel VIII durch Behandlung mit Zink in Eisessig oder einem Alkalimetallborhydrid übergeführt. Für diese Reaktion können die üblichen Bedingungen der Zink-Eisessig-Reduktion oder der Alkalimetallborhydrid-Reduktion verwendet werden. Ein bevorzugtes Alkalimetallborhydrid ist Natriumborhydrid.

Für die Umsetzung von Verbindungen der Formel VIII in Verbindungen der Formel IX können die üblichen Bedingungen für die Umsetzung eines Amins mit einem Chlorameisensäureester verwendet werden. Im allgemeinen ist es bevorzugt, die Reaktion in einem inerten organischen Lösungsmittel durchzuführen. Hierfür kann jedes konventionelle inerte organische Lösungsmittel verwendet werden. Ein bevorzugtes Lösungsmittel ist Tetrahydrofuran. Bei der Reaktion sind Temperatur und Druck nicht kritisch, und man kann bei Raumtemperatur und Atmosphärendruck arbeiten.

Die Verbindungen der Formel IX werden in Verbindungen der Formel II durch Behandlung mit einem Reduktionsmittel umgewandelt. Für diese Umsetzung kann jedes konventionelle Reduktionsmittel, das einen Ester zum Aldehyd reduziert, verwendet werden. Bevorzugt sind Alkylaluminiumhydrid-Reduktionsmittel wie Diisobutylaluminiumhydrid. Für die Durchführung der Reaktion kommen die für solche Reduktionen üblichen Bedingungen in Betracht.

Bei der Umwandlung einer Verbindung der Formel II in eine Verbindung der Formel I, in der $R^1$ eine Gruppe $-C(R^2,R^3,R^4)$ darstellt, kann jeder übliche Säurekatalysator verwendet werden. Bevorzugte Säurekatalysatoren sind Kationenaustauscherharze wie aromatische Sulfonsäure-Kationenaustauscherharze, z. B. Polystyrol-sulfonsäureharze wie Amberlit®IR-120, Dowex® 50 und Amberlit® CG 120 (Rohm und Haas). Es können aber auch anorganische oder organische Säuren verwendet werden, z. B. Schwefelsäure, Salzsäure, Bromwasserstoffsäure, Essigsäure und p-Toluolsulfonsäure. Bei der Reaktion ist es nicht notwendig ein inertes organisches Lösungsmittel zu verwenden, vorzugsweise führt man die Reaktion in Gegenwart von überschüssigem Methanol durch. Andererseits kann für die Reaktion gewünschtenfalls auch ein inertes organisches Lösungsmittel verwendet werden.

Reaktionstemperatur und Druck sind nicht kritisch. Die Reaktion kann bei Raumtemperatur und unter Atmosphärendruck durchgeführt werden, jedoch kann auch bei erhöhter oder herabgesetzter Temperatur gearbeitet werden. Gewünschtenfalls kann bei Temperaturen zwischen $-10°$ und $100°C$ gearbeitet werden, wobei Rückflußtemperatur des Reaktionsgemisches besonders bevorzugt ist.

Die Verbindung der Formel I, worin $R^1$ eine Gruppe $-C(R^2,R^3,R^4)$ ist kann in eine Verbindung der Formel I, in der $R^1$ Wasserstoff ist, durch Hydrogenolyse übergeführt werden. Hierfür kann jede konventionelle Hydrogenolysemethode eingesetzt werden. Vorzugsweise behandelt man die Ausgangsverbindungen mit Natrium in flüssigem Ammoniak. Andererseits kann auch katalytisch hydriert werden. Jede konventionelle Methode der katalytischen Hydrierung kann hierfür verwendet werden.

Die Verbindungen der Formel VI können aus Verbindungen der Formel

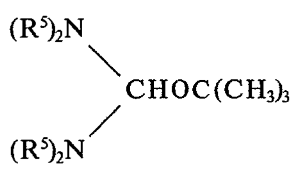

(IV)

und Propenylacetat hergestellt werden.

Bei dieser Reaktion sind Temperatur und Druck nicht kritisch. Man kann bei Raumtemperatur und Atmosphärendruck arbeiten. Andererseits kann bei erhöhter oder herabgesetzter Temperatur gearbeitet werden. Im allgemeinen wird die Reaktion vorzugsweise am Siedepunkt oder bei Rückflußtemperatur des Reaktionsgemisches durchgeführt. Jedoch können auch Temperaturen von $0-100°C$ angewandt werden. Obschon es bevorzugt ist, die Reaktion ohne Lösungsmittel durchzuführen, können auch konventionelle inerte organische Lösungsmittel gewünschtenfalls verwendet werden.

Die Verbindungen der Formel I sind als Zwischenprodukte bei der Synthese von Aminozuckern von Wert. Insbesondere können die Verbindungen der Formel I in den Aminozucker Methyl-L-daunosaminid (1-O-Methyl-L-daunosamin), eine Verbindung der Formel

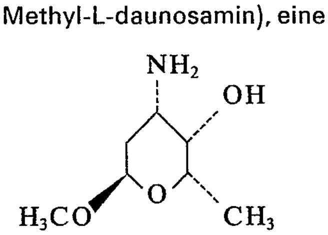

(A)

umgewandelt werden, die ein bekanntes Mittel zur Kupplung mit Daunomycinon oder Adriamycinon darstellt, wobei man die natürlichen Antibiotika Daunomycin und Adriamycin erhält, vgl. Acramone et al., J. of Med. Chem., 1975, Vol. 18, No. 7, Seite 703 und Arcamone et al., Cancer Chemotherapy Rep., 6, 123 (1975). Gemäß den vorstehend erwähnten Publikationen wird Methyl-L-acosaminid der Formel

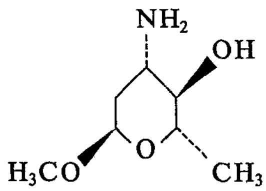

(B)

in C4'epi-Daunomycin und C4'epi-Adriamycin, bekannte Antitumor-Mittel, über eine Verbindung der Formel

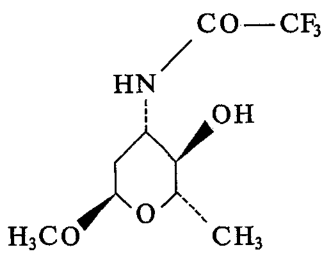

(B-1)

nach dem von Fuchs, J. Antibiotics, 32, 223 (1979) beschriebenen Verfahren übergeführt.

Verbindungen der Formel I, in der $R^1$ Wasserstoff ist, können in Verbindungen der Formel B durch basische Hydrolyse übergeführt werden. Jede konventionelle Methode der Basenhydrolyse kann hierfür verwendet werden, beispielsweise die Behandlung der Verbindung der Formel I mit einem Alkalimetallhydroxyd oder einem Erdalkalimetallhydroxyd. Die Reaktion wird üblicherweise in wäßrigem Medium bei Rückflußtemperatur ausgeführt.

Andererseits kann eine Verbindung der Formel I, in der $R^1$ Wasserstoff ist, in Verbindungen der Formel A über die Zwischenprodukte

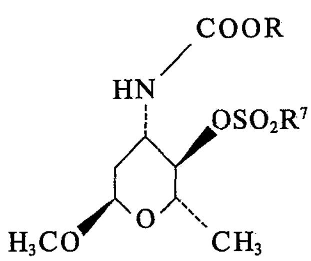

(X)

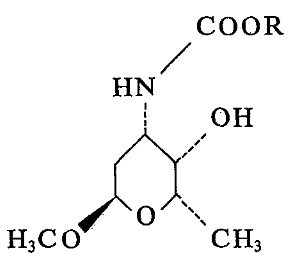

(XI)

in der R obige Bedeutung hat und $R^7$ $C_{1-7}$-Alkyl oder Aryl darstellt,
oder Racemate oder Enantiomerengemische davon übergeführt werden.

Die Verbindung der Formel I kann in eine Verbindung der Formel X durch Behandlung mit einem $C_{1-7}$-Alkylsulfonylhalogenid oder einem Arylsulfonylhalogenid übergeführt werden. Für diese Reaktion kann jedes konventionelle nieder-Alkylsulfonylhalogenid oder Arylsulfonylhalogenid die als Abgangsgruppe dienen, verwendet werden. Bevorzugt sind Methansulfonylchlorid und Toluolsulfonylchlorid. Diese Umsetzungen können unter den üblichen Methoden zur Umsetzung einer Hydroxygruppe mit einem Sulfonylhalogenid durchgeführt werden.

Die Verbindung der Formel X kann in eine Verbindung der Formel XI durch Behandlung mit einem Gemisch von Dimethylformamid und Wasser übergeführt werden. Hierbei sind Temperatur und Druck nicht kritisch. Die Reaktion kann bei Raumtemperatur und unter Atmosphärendruck durchgeführt

werden. Andererseits können auch erhöhte oder verminderte Drucke oder Temperaturen angewandt werden. Im allgemeinen ist es bevorzugt, die Reaktion bei Rückflußtemperatur des Reaktionsgemisches durchzuführen.Weiterhin ist es im allgemeinen bevorzugt, die Reaktion in Gegenwart eines Puffers, vorzugsweise einem Alkalimetallsalz einer nieder-Alkancarbonsäure wie Natriumacetat, durchzuführen.

Die Verbindung der Formel XI kann in eine Verbindung der Formel A durch Basenhydrolyse in der gleichen Weise umgewandelt werden, wie das für die Überführung der Verbindung der Formel I in eine Verbindung der Formel B beschrieben wurde.

Falls eine Verbindung der Formel III als Racemat hergestellt wird, fallen die nachfolgenden Zwischenprodukte ebenfalls als Racemat an. Diese racemischen Zwischenprodukte können im Verlaufe der Reaktionssequenz in die gewünschten Enantiomeren gespalten werden. Dies kann bei den Zwischenprodukten der Formeln A, B, I, VIII, IX und XI über die darin enthaltene freie Hydroxygruppe geschehen. Die Verbindungen können mit Dicarbonsäuren wie Bernsteinsäure verestert werden, und die resultierende freie Carbonsäuregruppe kann mit einem optisch aktiven Amin umgesetzt werden, wobei man diastereomere Salze erhält. Typische optisch aktive Amine sind Arginin, Brucin, $\alpha$-Methylbenzylamin, Chinin und Dehydrobietylamin. Die erhaltenen diastereomeren Salze können mit bekannten Mitteln, wie Kristallisation oder Chromatographie, getrennt werden. Nach der Trennung kann das Salz der gewünschten Enantiomeren-Konfiguration durch Hydrolyse wieder in die freie Hydroxyverbindung übergeführt werden.

Falls die Verbindung der Formel III in Form eines reinen Enantiomeren gebildet wird, wird die optische Konfiguration durch die nachfolgenden Reaktionsschritte beibehalten, so daß die Verbindungen der Formeln A und B in der vorstehend bezeichneten optischen Konfiguration erhalten werden.

Die Verbindung der Formel B kann über das Zwischenprodukt

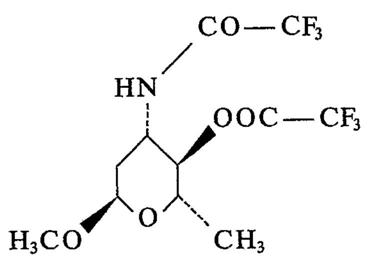

(XIII)

in das bekannte Kupplungsmittel zur Herstellung von C4'-Daunomycin oder C4'-Adriamcyin übergeführt werden.

Die Verbindung der Formel B wird zunächst in eine Verbindung der Formel XIII durch Umsetzung mit Trifluoressigsäureanhydrid umgewandelt. Die Reaktion wird in einem inerten organischen Lösungsmittel, vorzugsweise in einem ätherischen Lösungsmittel wie Äthyläther oder Tetrahydrofuran ausgeführt. Für die Reaktion können Temperaturen von 0°C bis Rückflußtemperatur des Reaktionsgemisches verwendet werden.

Die Verbindung der Formel XIII wird in einer Verbindung der Formel B-1 durch Behandlung mit Methanol übergeführt. Hierbei wirkt das Methanol als Lösungsmittel für die Verbindung der Formel XIII. Bei dieser Reaktion sind Temperatur und Druck nicht kritisch. Die Reaktion kann bei Raumtemperatur und Atmosphärendruck durchgeführt werden, im allgemeinen arbeitet man bei Temperaturen zwischen 0 und 50°C.

Die Erfindung wird durch die nachfolgenden Beispiele weiter erläutert.

## Beispiel 1

Eine Lösung von 20 g trans-Propenylacetat und 101 g Bis(dimethylamino)-tert.-butoxymethan wurde unter Argon 16 Stunden auf 50° erhitzt. Das überschüssige Reagens wurde durch Vakuumdestillation (0,7 Torr, 50° Badtemperatur) entfernt. Der Rückstand wurde rasch auf 300 g Silicagel 60 chromatographiert, wobei mit Äthylacetat/Hexan (2 : 3 Volumenteile) eluiert wurde. Man erhielt 28,2 g trans-3-(Dimethylamino)-2-propensäure-trans-propenylester, der ohne weitere Reinigung im nächsten Reaktionsschritt Verwendung fand. Dieses Material wurde zur Entfernung der gelben Farbe bei 40—50° und 0,2 Torr sublimiert. Ein Analysenpräparat wurde durch Umkristallisation aus Pentan erhalten, Smp. 48—48,5°.

## Beispiel 2

Ein Gemisch von 1,5695 g S(—)-N-Hydroxy-$\alpha$-methyl-benzylamin, 0,776 g trans-3-(Dimethylamino)-2-propensäure-trans-propenylester und 65 ml Xylol wurden 50 Minuten unter Inertgas zum Rückfluß

erhitzt. Das abgekühlte Reaktionsgemisch wurde filtriert und mit Dichlormethan gewaschen. Die Lösungsmittel wurden unter vermindertem Druck entfernt und das Produkt aus Diäthyläther kristallisiert. Man erhielt 0,4752 g (38%) [3S-[1(S*), 3β, 3aβ, 6aβ]]-Tetrahydro-3-methyl-1-(1-phenyläthyl)-6H-furo[3,2-c]isoxazol-5-on. Weitere 0,227 g (18%) Produkt wurden durch Chromatographie der Mutterlaugen auf 250 g Silicagel durch Elution mit Diäthyläther/Toluol (5 : 1 Volumenteile) erhalten. Ein Analysenpräparat wurde aus Diäthyläther umkristallisiert: Smp. 138—138,5°,$[\alpha]_D^{25}$ = +17,16 (c = 0,6876 in Chloroform).

## Beispiel 3

Zu einer Lösung von 0,9892 g [3S[1(S*)-3β,3aβ,6aβ]]-Tetrahydro-3-methyl-1-(1-phenyläthyl)-6H-furo[3,2-c]-isoxazol-5-on und 120 ml Essigsäure/Wasser (1 : 1 Volumenteile) wurden 3,7284 g Zinkstaub gegeben. Das Gemisch wurde 22½ Stunden unter Argonatmosphäre gerührt, danach filtriert und der Rückstand mit Wasser und darauf mit Äthylacetat gewaschen. Die Lösungsmittel wurden unter vermindertem Druck entfernt und der Rückstand in 400 ml Äthylacetat aufgenommen. Diese Lösung wurde mit 60 ml 2N Kaliumcarbonatlösung gewaschen und über Natriumsulfat getrocknet. Nach Filtrieren und Abdampfen des Lösungsmittels wurden 0,945 g Rohprodukt erhalten, das an 20 g Silicagel mit Äthylacetat chromatographiert wurde. Man erhielt 0,8913 g (89%) [4S-[4β, 4(S*), 5β,5-(S*)]]-Dihydro-5-(1-hydroxyäthyl)-4-[(1-phenyläthyl)amino]-2(3H)-furanon als Öl. Ein Analysenpräparat wurde aus Diäthyläther kristallisiert: Smp. 58—60°, $[\alpha]_D^{25}$ = —17,11° (c=0,9000 in Methanol).

## Beispiel 4

Zu einer Lösung von 10 g [4S-[4β,4(S*),5β,5(S*)]]-Dihydro-5-(1-hydroxyäthyl)-4-[(1-phenyläthyl)amino]-2(3H)-furanon in 400 ml Tetrahydrofuran wurden 800 ml 2N Natriumcarbonatlösung und 160 ml Chlorameisensäuremethylester gegeben. Das Gemisch wurde unter Kühlung 5 Stunden gerührt und darauf mit viermal 500 ml Äthylacetat extrahiert.Die vereinigten Extrakte wurden mit Wasser gewaschen und über Natriumsulfat getrocknet. Nach Entfernung des Lösungsmittel blieben 12,9 g Rückstand. Dieser wurde an 450 g Silicagel mit Äthylacetat chromatographiert. Man erhielt 9,82 g Produkt, aus dem nach Kristallisation aus Diäthyläther 6,94 g (54%) [4S[4β,4(S*),5β,5(S*)]]-Tetrahydro-5-(1-hydroxyäthyl)-2-oxo-N-(1-phenyläthyl)furan-4-aminsäuremethylester erhalten wurde. Ein Analysenpräparat wurde aus Äther umkristallisiert: Smp. 124,5°, $[\alpha]_D^{25}$ = —82,10 (c=1,378 in Chloroform).

## Beispiel 5

3,07 g [4S-[4β,4(S*),5β,5(S*)]]-Tetrahydro-5-(1-hydroxyäthyl-2-oxo-N-(1-phenyläthyl)-furan-4-carbaminsäuremethylester wurden unter Argon in 300 ml trockenem Tetrahydrofuran unter Trockeneis/Aceton-Kühlung mit 33 ml (ca. 0,05 Mol) Diisobutylaluminiumhydrid in Toluol (25%) behandelt. Nach 5 Stunden wurde das überschüssige Hydrid mit Methanol zerstört. Danach wurden 150 ml gesättigte wäßrige Natriumsulfatlösung und anschließend Kochsalzlösung zugesetzt. Das Gemisch wurde mit dreimal 500 ml Methylenchlorid extrahiert. Die Extrakte wurden mit Wasser gewaschen und über Natriumsulfat getrocknet. Nach Filtrieren und Abdampfen der Lösungsmittel unter vermindertem Druck wurden 3,347 g [4S-[4β,4(S*),5β,5(S*)]]-Tetrahydro-5-(1-hydroxyäthyl)-2-hydroxy-N-(1-phenyläthyl)-furan-4-carbaminsäuremethylester erhalten, die in dieser Form direkt im nächsten Schritt eingesetzt wurde.

## Beispiel 6

Der aus Beispiel 5 erhaltene Rückstand (3,347 g, ca. 0,010 Mol) wurde mit 5,75 g Amberlit CG 120 (200—400 Mesh) und 500 ml Methanol 4 Stunden gerührt. Das Gemisch wurde filtriert und das Lösungsmittel unter vermindertem Druck entfernt. Der Rückstand wurde an 150 g Silicagel chromatographiert, wobei mit Hexan/Äthylacetat (2 : 1 Volumenteile) eluiert wurde. Man erhielt 2,884 g (89%) eines Gemisches der Anomeren (ca. 4 : 1). Das überwiegende Anomer [2S-[2α,-3β, 4α, 4(S*), 6β]]-Tetrahydro-3-hydroxy-6-methoxy-2-methyl-4-N-(1-phenyläthyl)pyrancarbaminsäuremethylester wurde durch Mitteldruck-Flüssigchromatographie (Hexan/Äthyl-acetat 2 : 1 Volumenteile) isoliert. $[\alpha]_D^{25}$ = — 106,10° (c=0,9020 in Chloroform).

## Beispiel 7

Eine Lösung von 0,277 g [2S-[2α, 3β, 4α, 4(S*), 6β]]-Tetrahydro-3-hydroxy-6-methoxy-2-methyl-4-N-(1-phenyl-äthyl)pyrancarbaminsäuremethylester in 3 ml trockenem Tetrahydrofuran wurde auf

—78° gekühlt, worauf ca. 10 ml trockenes Ammoniak in das Reaktionsgefäß eindestilliert wurden. Darauf wurden 0,10 g Natriummetall zugesetzt, und das Gemisch wurde 3 Stunden gerührt. Nach Zugabe von festem Ammoniumchlorid wurde das Kühlbad entfernt und das Ammoniak in einem langsamen Argonstrom über Nacht verdampfen gelassen.Das Gemisch wurde in 20 ml gesättigter wäßriger Natriumbicarbonatlösung aufgenommen und die Lösung mit dreimal 50 ml Methylenchlorid extrahiert. Die vereinigten Methylenchloridextrakte wurden über Natriumsulfat getrocknet, filtriert und unter vermindertem Druck eingedampft, wobei 0,171 g Rohprodukt erhalten wurden. Dieses wurde aus Diäthyläther kristallisiert und lieferte 0,142 g (75%) [2S-(2α, 3β, 4α, 6α)]-Tetrahydro-3-hydroxy-6-methoxy-2-methyl-2H-pyran-4-carbaminsäuremethylester. Ein Analysenpräparat wurde aus Diäthyläther umkristallisiert: Smp. 142,5—143°, $[\alpha]_D^{25}$ = —162,64° (c=0,7071in Chloroform).

Beispiel A und B illustrieren die Umwandlung der in Beispiel 7 erhaltenen Verbindung in Methyl-L-acosaminid und Methyl-L-daunosaminid.

## Beispiel A

Eine Lösung von 0,020 g [2S-(2α,3β,4α,6β)]-Tetrahydro-3-hydroxy-6-methoxy-2-methyl-2H-pyran-4-carbaminsäuremethylester, 10 ml Wasser, 5 ml Methanol und 0,063 g Bariumhydroxyd-octahydrat wurden unter Argon 5 Stunden zum Rückfluß erhitzt. Danach wurden weitere 0,12 g Bariumhydroxydoctahydrat zugesetzt, und das Gemisch wurde 24 Stunden weiter erhitzt. Darauf wurde festes Trokkeneis zu dem gekühlten Reaktionsgemisch gegeben. Die Lösung wurde über Diatomeenerde filtriert und die Lösungsmittel unter vermindertem Druck entfernt. Der Rückstand wurde in Wasser aufgenommen und über ein AG-1-X4 (OH-Form) Ionenaustauscherharz (ein anionisches Polystyrol-Austauscherharz mit quaternären Ammoniumgruppen gegeben. Die Lösungsmittel wurden unter vermindertem Druck entfernt. Der Rückstand wurde in einen Destillationsapparat sublimiert und lieferte 0,0080 g (54%) Methyl-3-amino-2,3,6-trideoxy-α-L-arabinohexopyranosid (Methyl-L-acosaminid). Smp. 129,5—130,5° (Lit.: S.K. Gupta, Carbohydrate Res., 37, 38 (1974), Smp. 132—133°). Eine Schmelzpunkt-Depression wurde nicht beobachtet. NMR- und Massenspektrum waren identisch mit authentischem Material. $[\alpha_D^{25}$ = —140,38° (c=0,2251 in Methanol), Lit.: S.K. Gupta, Carbohydrate Res., 37, 38 (1974) = 1— 145,1° (=,61 in Methanol).

## Beispiel B

Einer Lösung von 0,3009 g [2S-(2α,3β,4α,6β)]-Tetrahydro-3-hydroxy-6-methoxy-2-methyl-2H-pyran-4-yl]carbaminsäuremethylester in 30 ml trockenem Pyridin wurde auf 0° gekühlt und mit 0,6 ml Methansulfonylchlorid versetzt. Nach 2$^1/_2$ Stunden wurde Eis zugesetzt. 5 Minuten später wurden 20 ml gesättigte wäßrige Natriumcarbonatlösung zugesetzt und das Gemisch mit dreimal 100 ml Methylenchlorid extrahiert. Die vereinigten Extrakte wurden über Natriumsulfat getrocknet, filtriert und eingeengt. Man erhielt 0,365 g Rohprodukt, das aus Diäthyläther umkristallisiert wurde und 0,2800 g (69%) [2S-(2α,3β,4α,6β)]-Tetrahydro-6-methoxy-2-methyl-3-[(methylsulfonyl)oxy]-2H-pyran-4-yl]carbaminsäuremethylester in einer ersten Fraktion lieferte. Ein Analysenpräparat wurde aus Äther umkristallisiert. Smp. 141—142°, $[\alpha_D^{25}$ = —109,50° (c=0,9717 in Chloroform).

Eine Lösung von 0,1189 g [2S-(2α,3β,4α,6β)]-[Tetrahydro-6-methoxy-2-methyl-3-[(methylsulfonyl)oxy]-2H-pyran-4-yl]carbaminsäuremethylester,0,320 g wasserfreies Natriumacetat und 40 ml 65% wäßriges Dimethylformamid wurden unter Argon 42$^1/_2$ Stunden erhitzt (105° Badtemperatur). Die Lösungsmittel wurden unter vermindertem Druck entfernt und der Rückstand in 20 ml gesättigter wäßriger Natriumbicarbonatlösung aufgenommen. Die Lösung wurde mit dreimal 60 ml Methylenchlorid extrahiert und die Extrakte getrocknet, filtriert und unter vermindertem Druck eingedampft. Man erhielt 0,081 g Rohprodukt, das an 50 g Silicagel unter Elution mit Äthylacetat chromatographiert wurde. 0,0435 g [2S-(2α,3α,4α,6β)]-(Tetrahydro-3-hydroxy-6-methoxy-2-methyl-2H-pyran-4-yl-carbaminsäuremethylester wurden erhalten (50%). Ein Analysenpräparat wurde aus Diäthyläther/Pentan umkristallisiert: Smp. 90—90,5°.

0,151 g [2S-(2α,3α,4α,6β)]-Tetrahydro-3-hydroxy-6-methoxy-2-methyl-2H-pyran-4-carbaminsäuremethylester wurden unter Argonatmosphäre mit 0,10 g Bariumhydroxyd-octahydrat, 10 ml Wasser und 5 ml Methanol 12 Stunden zum Ruckfluß erhitzt (Badtemperatur 110°) und dann 8 Stunden gerührt. Die Lösungsmittel wurden unter vermindertem Druck entfernt, der Rückstand in Methanol aufgenommen und filtriert. Die Lösungsmittel wurden unter vermindertem Druck entfernt und der Rückstand mit zweimal 40 ml Diäthyläther gekocht und dekantiert. Nach Entfernung der Lösungsmittel wurden 0,129 g Rohprodukt erhalten, das über eine Säule mit 2 ml AG-1-X4 Ionenaustauscherharz (OH-Form) mit Wasser als Lösungsmittel gegeben wurde. Nach Abdampfen des Lösungsmittels wurden 0,0051 g Methyl-3-amino-2,3,6-trideoxy-α-L-lyxohexopyranosid (Methyl-L-daunosaminid) erhalten (46%). Dieses Produkt zeigte dasselbe NMR-Spektrum wie authentisches Material. Das Produkt wurde bei ca. 60° unter 0,05 Torr sublimiert und zeigte einen Schmelzpunkt von104—106°, der im Gemisch mit authentischem Material (Literatur: Chem. Commun., 973 (1967), Smp. 109—110°) keine Schmelzpunkt-Depression zeigte.

**Patentanspruch**

Verfahren zur Herstellung von Verbindungen der allgemeinen Formel

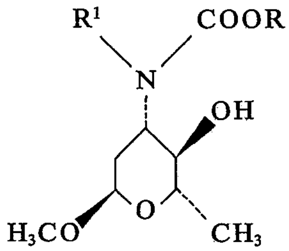

(I)

worin

R $C_{1-7}$-Alkyl,
$R^1$ Wasserstoff oder eine Gruppe $-C(R^2,R^3,R^4)$;
$R^2$ und $R^4$ Wasserstoff, Aryl, Aralkyl oder $C_{1-7}$-Alkyl und
$R^3$ Aryl ist,

deren Enantiomeren und Racemate, dadurch gekennzeichnet, daß man eine Verbindung der Formel

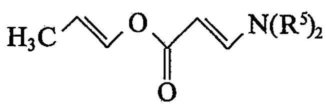

(VI)

worin $R^5$ $C_{1-7}$-Alkyl ist,
mit einer Verbindung der Formel

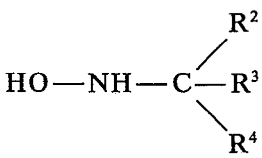

(VII)

worin $R^2$, $R^3$ und $R^4$ die oben angegebene Bedeutung besitzen,
oder einem Salz davon zu einer Verbindung der Formel

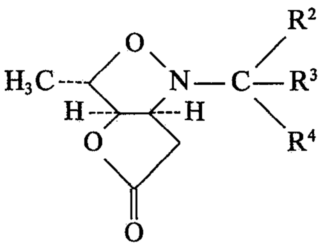

(III)

umsetzt, die man durch Behandlung mit einem geeigneten Reduktionsmittel zu einer Verbindung der Formel

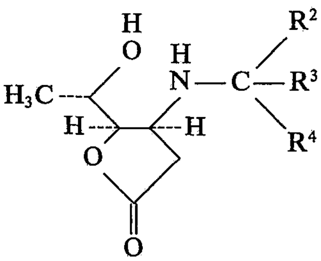

(VIII)

reduziert, welche mit einem Halogenameisensäureester der Formel

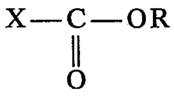

worin

X Halogen ist und
R $C_{1-7}$-Alkyl bedeutet,

in eine Verbindung der Formel

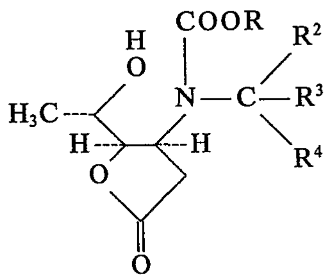

(IX)

übergeführt wird, die ihrerseits durch Behandlung mit einem Reduktionsmittel in eine Verbindung der Formel

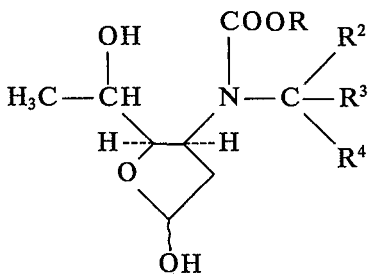

(II)

umgewandelt wird, die man sodann mit Methanol in Gegenwart eines Säurekatalysators zu einer Verbindung der allgemeinen Formel I, in der $R^1$ eine Gruppe $-C(R^2,R^3,R^4)$, darstellt oder einem Enantiomer oder dem Racemat davon umsetzt und gewünschtenfalls die so erhaltene Verbindung hydrogenolysiert, um eine Verbindung der allgemeinen Formel I oder ein Enantiomer oder Racemat davon zu erhalten, in der $R^1$ Wasserstoff ist.

## Claims

A process for the manufacture of compounds of the general formula

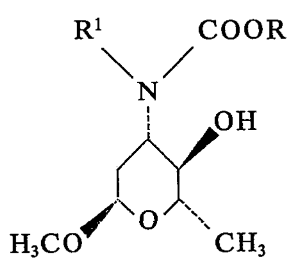

(I)

wherein

R is $C_{1-7}$-alkyl,
$R^1$ is hydrogen or a group $-(R^2, R^3, R^4)$;
$R^2$ and $R^4$ are hydrogen, aryl, aralkyl or $C_{1-7}$-alkyl and
$R^3$ is aryl,

their enantiomers and racemates, characterized by reacting a compound of the formula

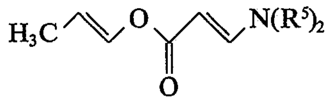

(VI)

wherein $R^5$ is $C_{1-7}$-alkyl,
with a compound of the formula

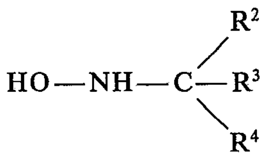

(VII)

wherein $R^2$, $R^3$ and $R^4$ have the significance given above,
or a salt thereof to give a compound of the formula

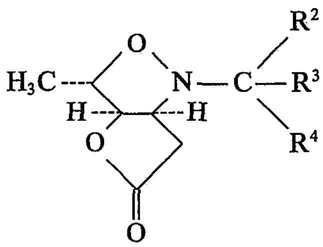

(III)

which is reduced by treatment with a suitable reduction agent to give a compound of the formula

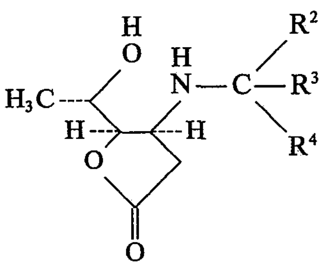

(VIII)

which is converted with a haloformic acid ester of the formula

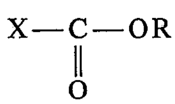

wherein

X is halogen and
R signifies $C_{1-7}$-alkyl,

into a compound of the formula

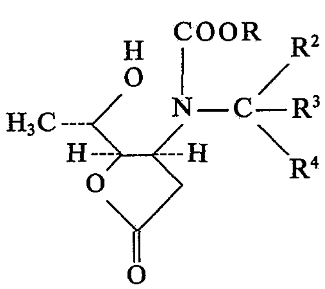

(IX)

which in turn is converted by treatment with a reduction agent into a compound of the formula

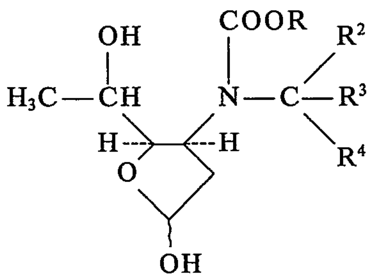

(II)

which is then reacted with methanol in the presence of an acid catalyst to give a compound of general formula I in which $R^1$ represents a group $-C(R^2, R^3, R^4)$ or an enantiomer or the racemate thereof and, if desired, the thus-obtained compound is hydrogenolyzed in order to obtain a compound of general formula I or an enantiomer or racemate thereof in which $R^1$ is hydrogen.

**Revendication**

Procédé de préparation de composés de formule générale:

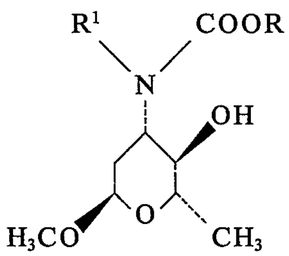

(I)

dans laquelle:

| | |
|---|---|
| R | représente un groupe alkyle en $C_1-C_7$, |
| $R^1$ | représente l'hydrogène ou un groupe $-C(R^2, R^3, R^4)$, |
| $R^2$ et $R^4$ | représentent l'hydrogène, des groupes aryle, aralkyle ou alkyle en $C_1$ à $C_7$ et |
| $R^3$ | représente un groupe aryle, |

de leurs énantiomères et racémates, caractérisé en ce que l'on fait réagir un composé de formule:

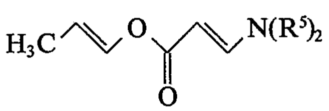

(VI)

dans laquelle $R^5$ représente un groupe alkyle en $C_1-C_7$,
avec un composé de formule:

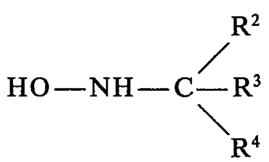

(VII)

dans la quelle $R^2$, $R^3$ et $R^4$ ont les significations indiquées ci-dessus,

ou un sel d'un tel composé, la réaction donnant un composé de formule:

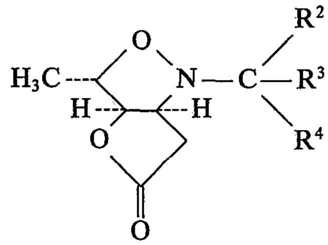

(III)

qu'on réduit par traitement à l'aide d'un agent réducteur approprié en un composé de formule:

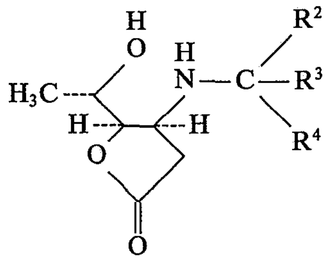

(VIII)

lequel est converti à l'aide d'un ester d'acide halogénoformique de formule:

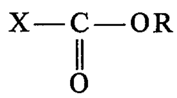

dans laquelle:

X représente un halogène et
R un groupe alkyle en $C_1-C_7$,

en un composé de formule:

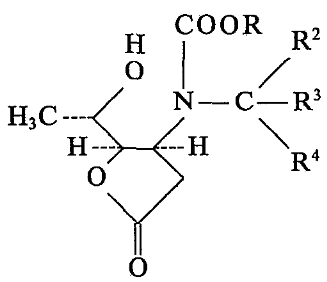

(IX)

lui-même converti par traitement à l'aide d'un agent réducteur en un composé de formule:

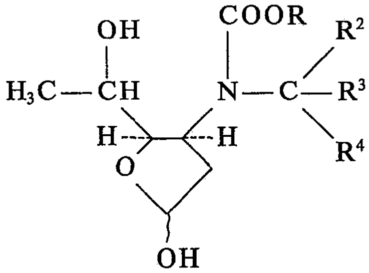

(II)

qu'on convertit ensuite à l'aide du méthanol en présence d'un catalyseur acide en un composé de formule générale (I) dans laquelle $R^1$ représente un groupe $-C(R^2, R^3, R^4)$ ou un énantiomère ou le racémate de ce composé qui, si on le désire, est soumis à hydrogénolyse pour obtention d'un composé de formule générale (I) ou d'un énantiomère ou racémate de ce composé dans lequel $R^1$ représente l'hydrogène.